# EUROPÄISCHE PATENTSCHRIFT

(11) **EP 2 908 908 B1**
(45) Veröffentlichungstag und Bekanntmachung des Hinweises auf die Patenterteilung: **25.01.2017**
(21) Anmeldenummer: 13828906.1
(22) Anmeldetag: 16.10.2013
(51) Int. Cl.: A61N 1/378

(54) **SPULE ZUR INDUKTIVEN TRANSKUTANEN ENERGIE- UND/ODER DATENÜBERTRAGUNG FÜR AKTIVE MEDIZINISCHE IMPLANTATE**
BOBBIN FOR INDUCTIVE TRANSCUTANEOUS ENERGY AND/OR DATA TRANSMISSION FOR ACTIVE MEDICAL IMPLANTS
BOBINE POUR LA TRANSMISSION TRANSCUTANÉE INDUCTIVE D'ÉNERGIE ET/OU DE DONNÉES POUR UN IMPLANT MÉDICAL ACTIF

(30) Priorität: 16.10.2012 DE 202012009867 U
(43) Veröffentlichungstag der Anmeldung: 26.08.2015
(73) Patentinhaber: em-tec GmbH, 86923 Finning (DE)
(72) Erfinder: HUBER, Elmar, 86916 Kaufering (DE)
(74) Vertreter: Castell, Klaus
(86) Internationale Anmeldenummer: PCT/DE2013/000601
(87) Internationale Veröffentlichungsnummer: WO 2014/059962

(56) Entgegenhaltungen:
- GB-A- 430 672
- Mike Tuggle: "Mike Tuggle's Re-Useable Spiderweb Coil Form", , 8. August 2003 (2003-08-08), XP002722278, Gefunden im Internet: URL:https://web.archive.org/web/2003100818 5824/http://www.crystalradio.net/spiderweb /index.shtml [gefunden am 2014-03-25]

## Beschreibung

Die Erfindung betrifft eine Spule zur induktiven transkutanen Energie- und/oder Datenübertragung für aktive medizinische Implantate, wobei Spulenwicklungen mit einem Leiter ausgeführt sind und in Spiralform um ein Zentrum verlaufen.

Spulen zur induktiven transkutanen Energieversorgung aktiver medizinischer Implantate sind aus dem Stand der Technik zahlreich bekannt.

Eine Spule nach der Präambel von Anspruch 1 ist aus XP002722278 bekannt.

Die transkutane kabellose Energieversorgung medizinischer Implantate (wie z. B. Blutpumpen, Herzunterstützungssysteme oder Kunstherzen) geschieht vor allem auf dem Wege der magnetischen Induktion mittels mindestens einer von Wechselstrom durchflossenen Primärspule (außerhalb des Körpers) und mindestens einer Sekundärspule (innerhalb des Körpers), die aufgrund der Induktionswirkung des von der Primärspule erzeugten magnetischen Wechselfelds ebenfalls von Wechselstrom durchflossenen wird. Die zu dieser induktiven transkutanen Energieübertragung verwendeten Spulen bestehen üblicherweise aus spiralförmig gewickelten, elektrische isolierten Kupferdrähten oder Kupferlitzen, bei denen die Leiter (Drähte oder Litzen) einer Wicklungswindung jeweils parallel zu den Leitern der Nachbarwindungen liegen. Zur Erzielung möglichst hoher Induktivitäten liegen die Windungen eng aneinander und zur möglichst guten Abfuhr der durch den ohmschen Drahtverluste erzeugten Wärme sind diese Spulen stets oblat zur Wicklungsachse ausgelegt (z. B. WO 2009/029977 A1, insbesondere die dortige Figur 18).

Gerade bei auf hohe Leistungen im Bereich bis ca. 50 Watt ausgelegten Systemen wie z. B. zur Versorgung von Blutpumpen, Herzunterstützungssystemen oder Kunstherzen treten auf der Primär- wie auf der Sekundärseite hohe Ströme auf, die einerseits die bereits erwähnte Verlustwärme verursacht und andererseits für das merkliche Auftreten des sogenannten Proximity-Effekts verantwortlich sind.

Unter dem Begriff Proximity-Effekt wird die Stromeinschnürung in sehr nahe benachbarten, von Wechselströmen durchflossenen Leitern verstanden. Die Stromeinschnürung wird durch die gegenseitige Induktion von Wirbelströmen hervorgerufen.

Damit tritt bei den nach dem Stand der Technik üblichen Spulen zur induktiven transkutanen Energieübertragung besonders im Falle hoher übertragener Leistungen folgendes Problem auf: Zum einen sollen die Spulen möglichst oblat ausgelegt sein, um bei gegebenem Umfang eine möglichst hohe Induktivität und eine möglichst gute Wärmeableitung zu erzielen. Dies bedingt beim üblichen Spulenaufbau, dass die Leiter aller Wicklungen aufgrund der parallelen Wicklungsführung über die gesamte Leiterlänge so eng wie möglich an ihren Nachbarwicklungen liegen.

Somit entsteht zum anderen ein sehr ausgeprägter Proximity-Effekt, der wiederum bedingt durch die Stromverschnürung zu einem erhöhten effektiven Leitungswiderstand und dadurch zu erhöhter Verlustwärme führt.

Ein weiteres Problem herkömmlicher oblater, eng spiralförmig gewickelter Spulen zur induktiven transkutanen Übertragung hoher Leistungen ist die Fixierung der Leiterenden, also der Teil des zur Spule gewickelten Leiters (Draht oder Litze), der im Innern und am Rand der Spule zum Zwecke des elektrischen Anschlusses an weitere elektronische Bauteile verbleiben muss. Üblicherweise werden diese Leiterenden durch Klebungen oder mechanische Hilfsmittel mit der Spule so verbunden, dass ein ungewolltes Abwickeln der Spule verhindert wird.

Zudem sind die üblichen oblaten, eng spiralförmig gewickelten Spulen zur induktiven transkutanen Übertragung hoher Leistungen sehr starr gegenüber Biegungen senkrecht zur Wickelebene und dadurch für eine Anwendung als Implantat, das einer Körperstelle möglichst gut angepasst werden soll, nicht optimal.

Aufgabe der vorliegenden Erfindung ist es, solche aus dem Stand der Technik bekannten Spulen zu verbessern.

Diese Aufgabe wird nach einem ersten Aspekt der Erfindung gelöst durch eine Spule zur induktiven transkutanen Energie- und/oder Datenübertragung für aktive medizinische Implantate, wobei Spulenwicklungen mit einem Leiter ausgeführt sind und in Spiralform um ein Zentrum verlaufen, wobei zwischen zwei benachbarten vollständigen Wicklungen n Kreuzungspunkte existieren, die eine Wicklungsebene definieren, wobei n eine ungerade Zahl > 3 ist und die Lage des gewickelten Leiters hinsichtlich der Wicklungsebene vor und nach dem Kreuzungspunkt relativ zur Wicklungsebene zwischen einer niedrigen Position unterhalb der Wicklungsebene und einer hohen Position oberhalb der Wicklungsebene alterniert und die Spule eine Umhüllung aus biokompatiblen Material aufweist.

Da n eine ungerade Zahl, ist dies gleichbedeutend mit dem Umstand, dass auch die Zustände zweier benachbarter Wicklungen zwischen ihren gemeinsamen Kreuzungspunkten radial zwischen einer niedrigen Position und einer hohen Position alternieren.

Mit einer solchen Spule ist es möglich, eine für die transkutane Übertragung hoher Leistungen geeignete Spule zu schaffen, die bei gegebenem Durchmesser und geringer Ausdehnung entlang der Wickelachse (also unter weitestgehender Beibehaltung der oblaten Ausführung) einen möglichst geringen Proximity-Effekt aufweist und gleichzeitig eine sehr gute Verlustwärmeabfuhr ermöglicht, durch ihre spezielle Bauform die Fixierung der Leiterenden auf einfache Art ermöglicht, und gegenüber Biegungen senkrecht zur Wickelebene flexibel ist und so der Körperstelle an der sie implantiert werden soll möglichst gut angepasst werden kann.

Von Vorteil ist, wenn die Spule eine Leerlaufspulengüte von 200 - 250 aufweist. Eine hohe Spulengüte Q ist Voraussetzung für hohe Effizienz bei gleichzeitig geringerer Wärmeentwicklung bei hohen Leistungen. Die Spule kann in einem Frequenzbereich von 50 - 250 Kilohertz, bevorzugterweise im Bereich von 100 - 200 Kilohertz betrieben werden.

Alternativ oder kumulativ zu einer transkutanen Energieübertragung ist eine Datenübertragung über die Spulen selbst, insbesondere eine unidirektionale Datenübertragung möglich. Alternativ oder kumulativ kann eine getrennte Telemetrieeinheit zur bidirektionalen und/oder unidirektionalen Datenübertragung vorgesehen sein.

Zudem kann die Spiralform erst mit einem bestimmten Abstand vom Zentrum beginnen.

Von Vorteil ist wenn radial benachbarte Kreuzungspunkte bezüglich des Zentrums stets den gleichen Winkel einschließen. So liegen diese Kreuzungspunkte auf einer Geraden, die ins Spulenzentrum führt.

Weiter von Vorteil ist, wenn die Wicklungsebene eine gekrümmte Ebene ist. So ist eine Anpassung des Implantats an eine bestimmte Körperstelle möglich. Dies erhöht den Komfort des Implantatträgers.

Weiter von Vorteil ist, wenn an einer Stelle eines größten Abstandes des Leiters zur Wicklungsebene eine Leiteraußenseite einen Abstand von mindestens 0,5, insbesondere von mindestens 1,0 des Leitungsdurchmessers zur Wicklungsebene aufweist. Dadurch, dass nun der Leiter in einer bestimmten Windung mit sich selbst in den beiden benachbarten Windungen nur an den Kreuzungspunkten Berührungen hat, wird der Proximity-Effekt bedingt durch die starke Abnahme der magnetischen Wechselwirkung zwischen den Wicklungen, wobei der Abstand ihrer Oberflächen mit d bezeichnet sei, angenähert gemäß einer d⁻³-Abhängigkeit, deutlich herabgesetzt.

Die niedrige Position und hohe Position gegenüber der Wickelebene kann daher aufgrund der starken Abnahme der magnetischen Wechselwirkung insbesondere im Bereich des Radius des Leiters 1 liegen, sodass die zur Wärmeabfuhr vorteilhafte, weitestgehend oblate Gestalt der Spule beibehalten werden kann. Wärmemessungen und Thermographie-Messungen haben gezeigt, dass bei entsprechend gewickelten Spulen durch den verminderten Proximity-Effekt die Verlustwärmeerzeugung geringer ausfällt und die Verlustwärmeabfuhr besser erfolgt, als bei vergleichbaren herkömmlichen Spulen. Auch geringere Abstände sind bereits vorteilhaft, noch größere Abstände sind denkbar.

Durch das Alternieren zwischen der niedrigen und der hohen Position können Hohlräume entstehen, die durchgängig von der Spulenmitte radial nach außen verlaufen. Diese ermöglichen verschiedenste Nutzungen dieser Hohlräume.

Insbesondere kann ein Leiterende durch einen Hohlraum geführt sein. So wird es unnötig, dieses Leiterende durch Klebungen oder mechanische Hilfsmittel zu fixieren. Zudem kann das Ende elegant im Hohlraum geführt werden.

Besonders von Vorteil ist es daher, wenn ein inneres Leiterende durch einen Hohlraum nach außen geführt ist. Dieses trägt zu einer flachen Ausgestaltung der Spule bei, da das innere Leiterende im Hohlraum geführt werden kann.

Weiter von Vorteil ist, wenn durch einen Hohlraum ein Sensor, insbesondere ein Temperatursensor geführt ist. Dieser kann sowohl eingeschoben als auch eingeflochten werden. Auch mehrere Sensoren können vorgesehen werden. Diese können der Temperatur- und Leistungsüberwachung dienen. Insbesondere kann es sich um Temperatursensoren insbesondere Thermistoren oder Hallsonden handeln.

Auch eine Nutzung des Hohlraumes zur Einbringung einer Antenne erneut sowohl insbesondere durch Einschieben als auch Einflechten ist vorteilhaft. So kann im Hohlraum eine Antenne geführt sein. Dabei kann es sich insbesondere um eine Antenne zur Kommunikation, zum Beispiel um eine vertikal gestellte Rahmenantenne zur bidirektionalen Kommunikation handeln.

Des Weiteren kann in einem Hohlraum ein Stützelement geführt sein. Der Hohlraum kann so zum Einbringen von starren oder flexiblen Stützelementen wie Stützhaltern, Stiften, Stäbchen, Röhrchen genutzt werden. Diese können eventuell mit Führungsrillen ausgestattet sein. Sie dienen als Abstandshalter, Träger und als Litzenführung. Sie können aus unmagnetischen Metallen, insbesondere aus Aluminium oder aus thermisch leitfähigem Kunststoff oder Keramik für die Verbesserung des thermischen Wärmeausgleichs im Sinne einer thermischen Homogenisierung sowie aus temperaturbeständigen isolierenden Kunststoffen ausgeführt sein. Auch Kombinationen der Materialien sind möglich.

Weiter von Vorteil ist, wenn ein Stützelement gebogen ist. Mit gebogenen oder anders geformten Stützelementen insbesondere Stützhaltern in der Form von Stiften oder Trägern sind konkave, konvexe oder anders geformte Spulenvarianten herstellbar. So vorgeformte Spulen können sich einer Körperkontur besser anpassen. Durch Einbringen von einem oder mehreren Objekten wie ausgeführt, beispielsweise in Form von geraden oder gebogenen Stäben in einen oder mehrere Hohlräume kann eine definierte Verformung der Spule gegenüber der Wicklungsebene erzeugt werden. Insbesondere können die formgebenden Objekte jeweils eine bestimmte Krümmung aufweisen. Diese Krümmung kann von formgebendem Objekt zu formgebendem Objekt unterschiedlich sein. Durch geeignete Ausführung und Art der Objekte kann die Spule weiterhin eine gewisse Flexibilität gegenüber Biegung und senkrecht zur Wicklungsebene aufweisen. Durch die Einbringung der unterschiedlichen Elemente in diese Hohlräume oder die Verflechtung der Elemente mit den Wicklungen die diese Hohlräume begrenzen, können die verschiedenen Elemente gut eingebracht werden, ohne durch eine zusätzliche Fixierungseinrichtung gesichert zu sein. Handelt es sich bei den Elementen um Leiterenden, so ist ein gewolltes Abwickeln der Spule somit wirksam verhindert.

Weiter von Vorteil ist, wenn zwei Leiterenden am Spulenanschluss verdrillt sind. So werden unerwünschte Streufelder minimiert.

Bereits eine Wicklung hat hierbei einen positiven Effekt. Es sind jedoch auch zahlreiche Wicklungen möglich. Weiter von Vorteil ist, wenn die Spiralform als Außenkontur eine ovale Form, insbesondere ein Lemesches Oval, also eine Pflasterform oder eine elliptische Form aufweist. Weiter von Vorteil ist, wenn die Spule mehrere Wicklungsebenen aufweist. Dies ist beispielsweise bei einer Wabenspule der Fall. Insbesondere drei oder mehr Ebenen sind denkbar.

Die Kreuzungspunkte können fest miteinander verbunden sein. Auch eine freiliegende Spule ist denkbar. Diese ist besonders flexibel. Da lediglich ein Kontakt in den Kreuzungspunkten vorliegt, vermindert sich die Reibung der Leiteroberfläche zwischen den Wicklungen. Dies führt zu einer erhöhten Flexibilität der Spule gegenüber Biegungen senkrecht zur Wirkungsebene.

Durch eine Verbindung der Kreuzungspunkte kann jedoch die Steifigkeit der Spule beeinflusst werden. Dies kann durch Binden beispielsweise mit einem Faden, Kleben oder auch geeignete Harze oder Lacke geschehen. Eine flexible Spule kann beispielsweise nur an den zentralen Schnittpunkten, wie beispielsweise der letzten Windung geklebt werden. Damit können sich alle nicht verbundenen Stellen leicht gegeneinander verschieben, was der Spule eine flexiblere, weichere Eigenschaft gibt. Ist eine steifere oder starre Spule gewünscht, ergibt sich ein starres Gebilde, wenn alle Kreuzungspunkte miteinander verbunden oder die gesamte Spule in geeigneten Lack oder Kleber getaucht wird. Als Kleber eignet sich beispielsweise ZweiKomponenten-Kleber. Auch hier kann mit dem Mischungsverhältnis des Härters die Steifigkeit beeinflusst werden. Da Cyanatcrylat-Klebstoffe, wie sie in Sekundenklebern verwendet werden, auf Feuchtigkeit wie Wasser stark reagieren können, sind diese nicht für Spulen geeignet, welche später silikoniert werden sollen. Für implantierbare Spulen kommen insbesondere biokompatible Kleber mit einer USP-Klasse VI Zulassung, wie zum Beispiel der Silikonkleber Silupran 4200 oder ein zweistufiger Silikonierungsprozess in Frage. Damit können sehr elastische Verbindungen hergestellt werden.

Von Vorteil ist, wenn das biokompatible Material für die Umhüllung ein Silikon ist. Um eine Implantierung zu ermöglichen muss die Spule mit biokompatiblem Material hermetisch dicht in eine Umhüllung eingebettet werden. Von Vorteil ist dabei, wenn die Spule direkt in eine entsprechende Umhüllung eingeschlossen wird.

Das biokompatible Material kann auch ein anderer langzeit getesteter biokompatibler Kunststoff wie Polymere wie Polyurethan (PUR), PTFE, PEEK oder andere temperaturbeständige Thermoplaste. In der USP-Klasse VI (Implantations Tests nach ISO10993-6, intrakutaner Test, toxikologischer und zytotoxischer Test nach ISO 10993-5, Hämokompatibilität nach ISO10993-4) werden als Referenzmaterialien nach ISO10933-12 beispielsweise folgende Materialien insbesondere folgende Polymere genannt: PTFE Polytetrafluorethylen (Teflon) das sterilisierbar ist, insbesondere mit ETO und Autoclav, PEEK Polyetheretherketon, das ebenfalls sterilisierbar ist insbesondere mit Autoclav, FEP Fluorethylenpropylen, das ebenfalls sterilisierbar ist insbesondere mit ETO und Autoclav, ePTFE das implantierbar ist, eine Endothelisation unterstützt und antithrombogen ist, PFA Perfluoralkoxy sowie ETFE Ethylentetrafluaorethylen.

In dieser USP-Klasse werden systemische Einspritzung, ein intrakutaner Test und ein Implantationstest vorgenommen. Auch Mischungen dieser Materialien sowie verschiedene Schichten und Umhüllungen sind denkbar.

Vorteilhafterweise weist das biokompatible Material eine Shore-Härte-A von 20 bis 50 insbesondere von 20 bis 40 auf. Durch die Shore-Härte ist es möglich, die Flexibilität des Implantats zu variieren. Ein weicheres Implantat hat dabei den Vorteil, dass eine erhöhte Flexibilität z. B. als Reaktion auf Körperbewegungen vorhanden ist. Dies ist insbesondere für implantierte Spulen von Vorteil. Bei extrakorporalen Spulen können auch höhere Shore-Härten sinnvoll sein.

Weiter vorteilhaft ist, wenn die Spule mit Umhüllung eine Dicke von 10 mm vorzugweise von 5 bis 8 mm aufweist. Je dünner die Spule ist, desto angenehmer ist sie als Implantat zu tragen. Zwar sind auch größere Dicken denkbar, diese setzen den Komfort jedoch wesentlich herunter.

Weiter von Vorteil ist, wenn die Umhüllung eine Befestigungsvorrichtung aufweist. So ist es möglich, dass Implantat im Körper zu befestigen. Hierbei kann die Befestigungsvorrichtung eine Hülse oder ein steriles Band sein. Eine Öse dient bei der Implantation der Spule der chirurgischen Fixierung der Umhüllung zur Sicherung gegen eine ungewünschte Wanderung im Körper, indem eine Vernähung mit festem biologischem Gewebe vorgenommen wird. Wird diese Befestigungshilfe aufgrund der anatomischen Lage nicht benötigt, kann diese bei entsprechender Wahl des Umhüllungsmaterials vom Chirurgen mit einem Skalpellschnitt leicht entfernt werden. Eine konstruktive Alternative dazu ist es ein steriles Band beispielsweise in Form eines umlaufenden Ringes einzugießen.

Die Spule kann eine ferromagnetische Platte aufweisen. Diese kann in die Umhüllung eingegossen sein. Sie dient der Schirmung. Dabei wird man darauf achten, dass die ferromagnetische Platte vorzugsweise in der Größenordnung von 0,2 bis 2 mm liegt, um insbesondere das Implantat in der Gesamtdicke nicht unnötig zu vergrößern.

Vorteilhaft ist, wenn die Spule zwei Anzapfungen, eine oder keine Anzapfung aufweist.

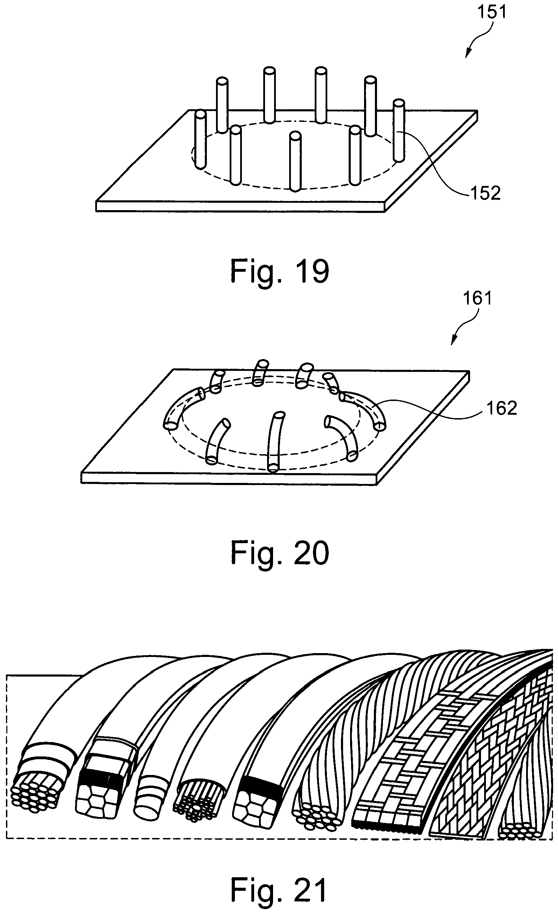

Weiter vorteilhaft ist, wenn der Leiter eine HF-Litze ist. Hochfrequenzlitzen (HF-Litzen) bestehen aus einzelnen isolierten Drähten, die zu dünnen Bündeln verflochten sind, mit einem typischen Durchmesser von 0,5 mm - 3 mm. Diese dienen bereits der Reduzierung von Verlusten durch beispielsweise den Skin- und Proximity-Effekt. Möglich sind beispielsweise Kupferlitzen. Alternativ können auch Silberlitzen verwendet werden, die eine erhöhte elektrische und thermische Leitfähigkeit und eine bessere Biokompatibilität aufweisen. Dabei kann eine Litze typischerweise einhundert bis zweihundertfünfzig Drähte mit jeweils 0,05 bis 0,1 mm Durchmesser aufweisen. Diese können beispielsweise mit Polyurethan (sogenannte ennamelled-insulated copper litz wires) und/oder einfach oder mehrfach umsponnen sein. Die Umspinnung kann aus Kunstseide, Mylar- oder anderen Kunststofffolien mit guten Isolationseigenschaften bestehen. Unterschiedliche Bündelungen und Isolierungsformen für HF-Litzen sind in Figur 21 zu sehen.

Ein zweiter Aspekt der Erfindung betrifft ein Spulenpaar zur induktiven transkutanen Übertragung von Leistung zur Energieversorgung aktiver medizinischer Implantate mit einer implantierbaren Sekundärspule und einer externen Primärspule, wobei eine der Spulen, insbesondere die implantierbare Sekundärspule eine erfindungsgemäße Spule ist. Ein solches Spulenpaar ermöglicht die transkutane Energieübertragung, optional kann eine Datenübertragung, insbesondere eine unidirektionale Datenübertragung vorgesehen sein.

Von Vorteil ist, wenn das Spulenpaar eine Ausrichthilfe aufweist. Um eine Verschiebung der Spulen zu vermeiden ist für den Benutzer hilfreich dass das TET-System eine Rückmeldung darüber liefert, wie gut die Spulen zentriert, d. h. wie gut die induktive Kopplung und damit die Energieübertragungseffizienz ist. Hier gibt es mindestens drei Lösungsmöglichkeiten:
Erstens kann eine Minimierung von Relativbewegungen durch Fixierung der Spulen zum Beispiel durch eine chirurgische Fixiermöglichkeit an Ösen, erfolgen.
Zweitens kann eine Ausrichtung durch zylindrische Scheibenmagnete, vorzugsweise in Form zylindrischer Ferritmagnete mit axialer Permanentmagnetisierung, vorzugsweise der Güte Y35, erfolgen. Ferritmagnetscheiben auf Ferritbasis (Composites) sind eine einfache Lösung den Patienten und Arzt bei der optimalen Positionierung der Spulen zu unterstützen. Die Magnetkräfte solcher typischerweise axialmagnetisierte Scheibenmagnete (so insbesondere mit einem Durchmesser von 20 mm) können über Distanzen von weit mehr als 10 mm deutlich wahrgenommen werden. Das zusätzliche Gewicht solcher Magnetscheiben beträgt zwischen 5 und 10 Gramm.
Drittens kann ein elektronisches Feedback mit Navigationshilfe vorgesehen sein. Als elektronisches Feedback kann die Auswertung und Rückmeldung der empfangenen Leistung oder die Höhe der Spannung auf der Sekundärseite herangezogen werden.

Weiter von Vorteil ist, wenn das Spulenpaar eine Sicherheitselektronik aufweist. Diese ermöglicht es, einen sicheren Betrieb des Spulenpaars bei Implantationen zu gewährleisten.

Bei der Sicherheitselektronik kann es sich um eine Fremdobjekterkennung und/oder eine analoge Testanfrage und/oder eine digitale Identifizierung und/oder einen Überstromschutz und/oder eine Spannungsbegrenzung und/oder eine Temperaturerfassung und/oder einen Hochspannungstransietenschutz (high-voltage surge stopper) und/oder einen ESD-Schutz und/oder eine Ausschaltmöglichkeit und/oder einen Standby-Modus des aktiven SynchronGleichrichters handeln.

Eine Fremdobjekterkennung (Foreign Object Detection FOD) funktioniert folgendermaßen: Um zu verhindern, dass während der induktiven Energieübertragung in einem elektrisch leitfähigen Fremdkörper eine Aufheizung durch Wirbelströme stattfindet, wird ständig die Sendeleistung mit der Empfangsleistung verglichen. Ergibt sich eine große Differenz deutet dies auf die Anwesenheit eines metallischen Fremdkörpers hin. Daraufhin muss die Energieübertragung vermindert oder abgeschaltet werden. Das aktive Implantat muss automatisch auf den internen Energiespeicher umschalten. Durch eine optische und akustische Alarmgenerierung wird der Patient angewiesen, den Fremdkörper zu entfernen. Nach Entfernen kann die Übertragung wieder fortgesetzt werden.

Unter einer analogen Testanfrage (Analog Ping) ist folgendes zu verstehen: Bei jedem Einschaltvorgang des Spulentreibers wird mit einem kurzen oszillatorischen Einschaltimpuls überprüft, ob ein adäquater Empfänger mit erwarteter Sekundärspuleninduktivität bereit ist. Hierzu wird die Rückantwort hervorgerufen durch die Gegeninduktivität ausgewertet. Falls nicht liegt ein Fehlerfall vor und eine entsprechende Alarmierung muss erfolgen.

Unter einer digitalen Identifizierung (Digital Ping) sei folgendes verstanden: Um ganz sicher zu gehen, dass kein unbekannter Empfänger eine ungewollte Energieübertragung hervorrufen kann, wird eine digitale Identifizierung der Empfangsspule(n) durchgeführt.

Des Weiteren können die bereits erwähnten Schutz- und Sicherheitsfunktionen auf der Sekundärseite zusätzlich alternativ oder kumulativ vorgesehen sein: Überstromschutz, Spannungsbegrenzung, Temperaturerfassung, Hochspannungstransietenschutz (High voltage surge stopper), ESD-Schutz, Ausschaltmöglichkeit und Standby des aktiven Synchrongleichrichts

Weiter von Vorteil ist, wenn das Spulenpaar Leistungen in der Größenordnung von 5 - 50 Watt, insbesondere von 5 - 40 Watt, insbesondere von 10 - 30 Watt überträgt. Dies ermöglicht eine ausreichende Energieübertragung für aktive medizinische Implantate.

Besonders vorteilhaft ist, wenn das Spulenpaar für eine Übertragung über eine Distanz von 5 - 40 mm, insbesondere von 5 *-* 15 mm, insbesondere von 10 - 15 mm geeignet ist. Dies sind die normalerweise in der transkutanen Energieversorgung auftretenden Distanzen, wobei eine geringe Distanz für den Betrieb des Systems vorteilhaft ist eine größere mögliche Abweichung jedoch eine erhöhte Sicherheit für den Implantatträger gewährleistet.

Ein dritter, unabhängiger Aspekt der Erfindung betrifft ein Spulensystem mit einem erfindungsgemäßen Spulenpaar, wobei es ein weiteres Spulenpaar aufweist. So können zwei unabhängige Leistungspaare realisiert werden. Das Spulensystem kann auch eine zweite implantierbare Sekundärspule, insbesondere eine erfindungsgemäße Spule aufweisen. So kann ein Strompfad für ein VAD und der andere zum Laden des internen Energiespeichers genutzt werden.

Ein vierter Aspekt der Erfindung betrifft ein aktives medizinisches Implantat, insbesondere ein Herzunterstützungssystem, insbesondere mit einem Leistungsbedarf von 5 - 50 Watt mit einer Spule, insbesondere einer erfindungsgemäßen Spule oder einem Spulenpaar, insbesondere einem erfindungsgemäßen Spulenpaar oder einem Spulensystem, insbesondere einem erfindungsgemäßen Spulensystem. Dieses kann jeweils alternativ oder kumulativ auftreten.

Ein letzter Aspekt der Erfindung betrifft ein Verfahren zur Herstellung eines Spulensystems.

Dabei kann es sich um ein Wickelverfahren handeln. Dieses kann die folgenden Schritte umfassen:
1) HF Litze auf die gegebene oder berechnete Länge unter doppeltem Zuschlag der gewünschten Anschlußlänge zuschneiden (typisch: 5 - 10 cm)
2) Die sauber abgeschnittenen Enden werden in einem bleifreien Tauchlötbad (abhängig von der Litze in der Regel bei > = 450°C) ca. 10 mm eingetaucht. Solange warten bis die Isolationslackschicht verdampft und die Enden verzinnt sind (ca. 5 Sekunden).
3) Empfehlung: DC Widerstandsmessung zur Qualitätssicherung
4) An einem Ende wird nach der Anschlußlänge am äußeren Speichenende mit Klebeband fixiert.
5) Die HF Litze wird zunächst an einer Speiche entlang von außen nach innen geführt.
6) Dann wird die Wickelvorrichtung nach rechts (für eine rechtsdrehende Spule) oder nach links (für eine linksdrehende Spule) gedreht. Während der Drehung wird die Litze unter möglichst konstantem Zug bei jeder Speiche alternierend nach oben oder unten (Zustände) geführt. Da die Anzahl der Speichen ungerade ist kommt man nach genau einer Umdrehung beim anderen Zustand (oben falls unten begonnen wurde oder umgekehrt) an.
7) Das Wickelwerkzeug wird manuell oder angetrieben von einem Schrittmotor immer weiter in die gewählte Drehrichtung weitergedreht.
8) Nach der nächsten vollständigen Umdrehung bei alternierendem Zustandswechsel von Speiche zu Speiche wird der Anfangszustand erreicht.
9) Zu beachten: Nach der zweiten Umdrehung sollte die Anschlußleitung automatisch im Hohlraum der Spule verlaufen. Ist dies nicht der Fall ist diese einzuschlaufen.
10) Auf diese Weise wird fortgefahren bis der gewünschte Außendurchmesser der Spule erreicht bzw. der Litzendraht zu Ende ist.
11) Fixieren des Endes an der gewünschten Austrittsstelle (in der Regel immer nach der vollen Umdrehung = Windungszahl)
12) Optionale Empfehlung: Ein Anschlussleiter an der Eintrittsstelle ggf. bis in den Hohlraum hinein mit Schrumpfschlauch nochmal isolieren.
13) Optionale Empfehlung: Leiter können zur Minimierung von Streu- und Störeinflüsse verdrillt werden (typischerweise reicht 1 Schlag pro cm aus)
14) Verbinden bzw. Verkleben der Kreuzungspunkte (siehe Beschreibung)
15) Speichen können nun aus dem Wickelwerkzeug herausgezogen werden.
16) Nach Herausziehen der Speichen ist Platz zum Einführen von etwaigen Sensoren (z. B. Thermistoren)

Auch eine vertikale Wickeltechnik ist denkbar:
Analog zur oben beschriebenen Wickeltechnik in horizontaler Lage kann die Wickelebene auch um 90° gedreht werden. Dies ergibt beispielsweise eine Wicklungsvorrichtung (Abbildung 25) mit der kurze zylindrische Luftspulen hergestellt werden können. Wegen Ihrer Höhe von vorzugsweise 10 bis 20 mm sind sie als transkutane Spulen weniger geeignet. Als externe Primärspule kann diese Wickeltechnik hingegen sehr wohl zum Einsatz kommen. Vorteil: Großer vom Feld durchdrungener Bereich, alle Windungen tragen nahezu den gleichen Beitrag zur elektrischen Flussdichte bei. In Abbildung 26 wird gezeigt wie auch geformte Spulenwicklungen möglich sind. Mit dieser Wickelvorrichtung kann eine konische Spule hergestellt werden, welche sich einer eventuellen Hautausbeulung anpassen könnte.

Eine so hergestellte Wicklung kann beispielsweise im Falle eines Silikongussverfahrens folgende Schritte umfassen:

Als Gussmaterial wird biokompatibles Silikon verwendet, das auf die gewünschte Shore-Härte angemischt wird. Um ein blasenfreies Gießen zu ermöglichen, wird die Vakuumkammer entgast. Daraufhin wird eine Unterseite in einer Gussform, vorzugsweise in der Höhe von ungefähr einem Millimeter gegossen und zwei bis drei Stunden in einem Ofen bei 30° ausgehärtet. Gegebenenfalls wird eine magnetische Scheibe auf den Silikonboden gelegt. Ebenso wird die vorher hergestellte Wicklung aufgelegt. Die Anschlussstelle der Litzen wird mit Formbauknetmasse abgedichtet. Die mit dem Oberteil versehene Form wird mit Silikon ausgefüllt. Es erfolgt ein Entgasen in der Vakuumkammer. Der Aushärtprozess kann beispielsweise über Nacht erfolgen. Ein Entformen erfolgt gegebenenfalls mit Hilfe von Drucklufteinsatz. Anschließend kann die Form mit Isopropanol gereinigt werden.

Außer dem dargestellten Silikonprozess kann die Umhüllung der Spule auch mit temperaturbeständigen Thermoplastischen Elastomeren mit Hochdruckumspritzung geschehen [PCT/US2011/030136: WO2011/126791]. Die mit einer Hochdruckumspritzung entstehenden Probleme werden in der gleichen Patentschrift offengelegt.

Die Figur wird im Folgenden anhand von Ausführungsbeispiel unter Bezugnahme auf die Bezeichnung näher erläutert. Hierin zeigen
- Figur 1a: eine schematische Darstellung einer Wicklung für eine Spule ohne Trägerröhrchen
- Figur 1b: eine schematische Darstellung einer Wicklung für eine Spule mit einer Magnetscheibe
- Figur 1c: eine schematische Darstellung einer Seitenansicht einer Wicklung für eine Spule
- Figur 1d: eine schematische Darstellung einer Wicklung für eine Spule mit Trägerröhrchen und Magnetscheibe
- Figur 2a: eine schematische Darstellung einer 3D Ansicht einer Spule eingebettet in eine Silikonumhüllung
- Figur 2b: eine schematische Darstellung einer Seitenansicht einer Wicklung eingebettet in eine Silikonumhüllung
- Figur 3a: eine schematische Darstellung einer 3D Ansicht einer Spule mit einer Silikonumhüllung ohne Treiber oder Empfangselektronik
- Figur 3b: eine schematische Darstellung eines Ausschnitts, zeigend die Anschlussstelle der in Figur 3 dargestellten Spule mit Silikonumhüllung
- Figur 4a: eine schematische Darstellung einer Draufsicht auf eine Einzelspule mit Treiber oder Empfangselektronik
- Figur 4b: eine schematische Darstellung einer Seitenansicht einer Einzelspule mit Treiber oder Empfangselektronik
- Figur 4c: eine schematische Darstellung einer 3D Ansicht einer Einzelspule mit Treiber oder Empfangselektronik
- Figur 4d: eine weitere schematische Darstellung einer 3D Ansicht einer Einzelspule mit Treiber oder Empfangselektronik
- Figur 5a: eine schematische Darstellung einer 3D Ansicht einer Einzelspule mit Treiber oder Empfangselektronik
- Figur 5d: eine schematische Darstellung eines Ausschnitts, zeigend die Anschlussstelle zu der in Figur 5a dargestellten Einzelspule mit Treiber oder Empfangselektronik
- Figur 6a: eine schematische Darstellung einer Draufsicht auf eine größere externe Primärspule mit Treiberelektronik
- Figur 6b: eine schematische Darstellung einer Draufsicht auf eine kleine implantierbare Spule mit gleicher Elektronik
- Figur 7: eine schematische Darstellung einer Draufsicht eines Spulensystems
- Figur 8: eine schematische Darstellung einer Seitenansicht eines Spulensystems
- Figur 9: eine schematische Darstellung einer 3D Ansicht eines Spulensystems
- Figur 10a: eine schematische Darstellung der 3D Ansicht der Außenansicht eines Spulensystems

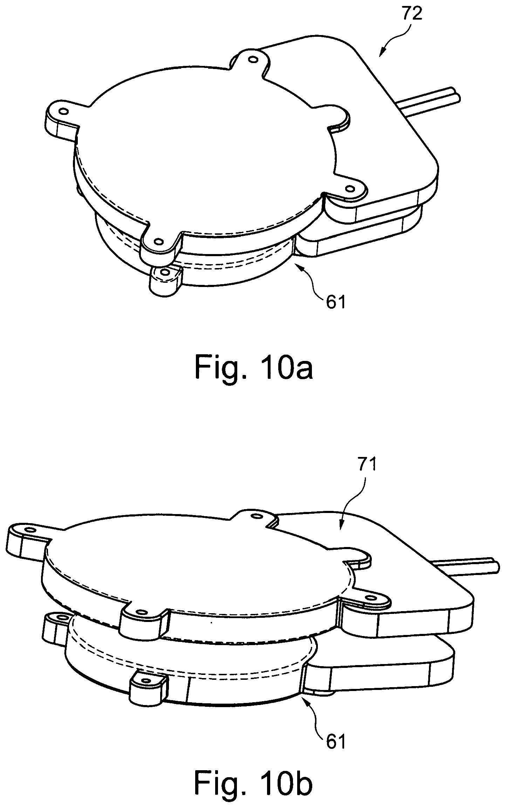

- Figur 10 b: eine weitere schematische Darstellung der 3D Ansicht der Außenansicht eines Spulensystems

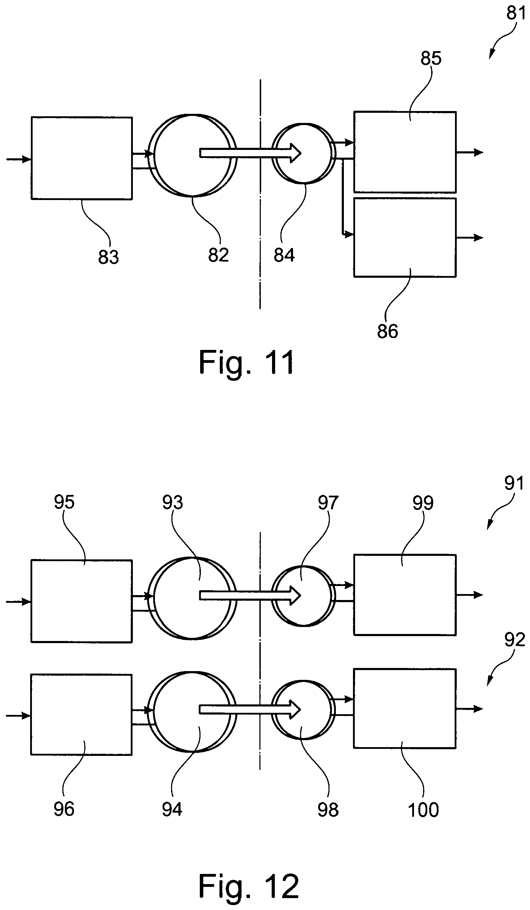

- Figur 11: eine schematische Darstellung eines Spulensystems mit einer Primärspule mit Treiber und einer Sekundärspule die zwei Verbraucher versorgt
- Figur 12: eine schematische Darstellung eines Spulensystems mit zwei unabhängigen Leistungspfaden

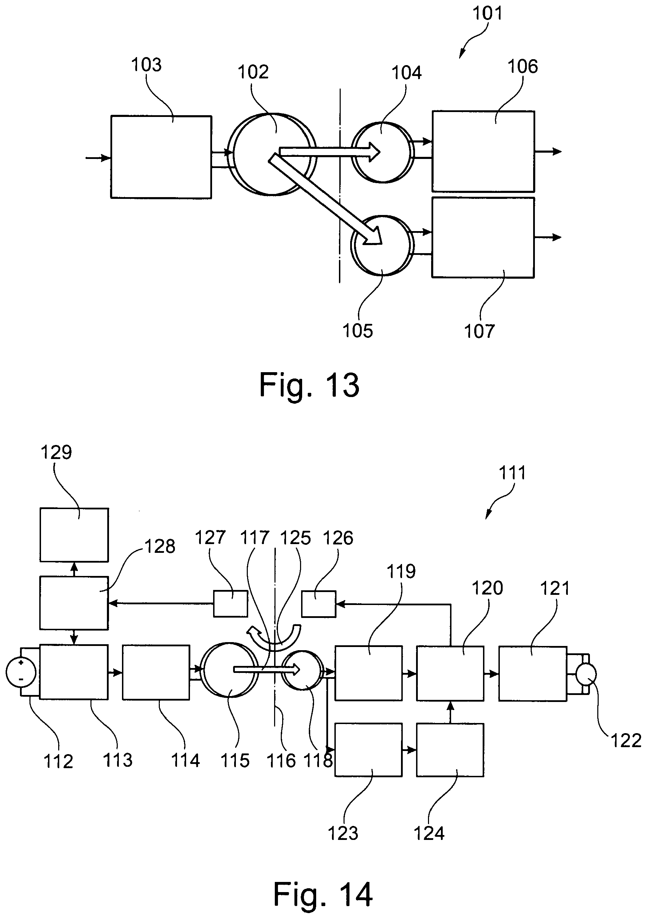

- Figur 13: eine schematische Darstellung eines Spulensystems in welchem eine Primärspule zwei Sekundärspulen betreibt
- Figur 14: eine schematische Darstellung eines transkutanen Energieübertragungssystems

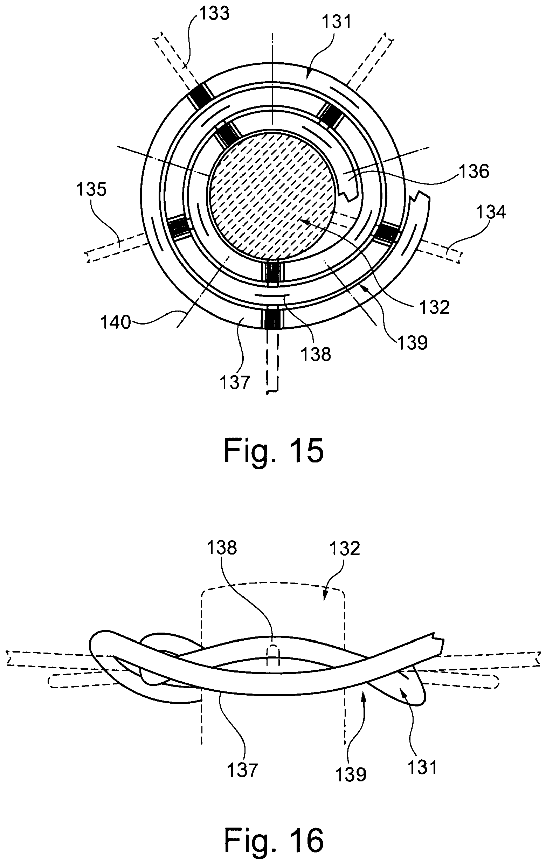

- Figur 15: eine schematische Darstellung einer Wickelvorrichtung mit Litzendraht in Draufsicht
- Figur 16: eine schematische Darstellung einer Wickelvorrichtung mit Litzendraht in Seitenansicht

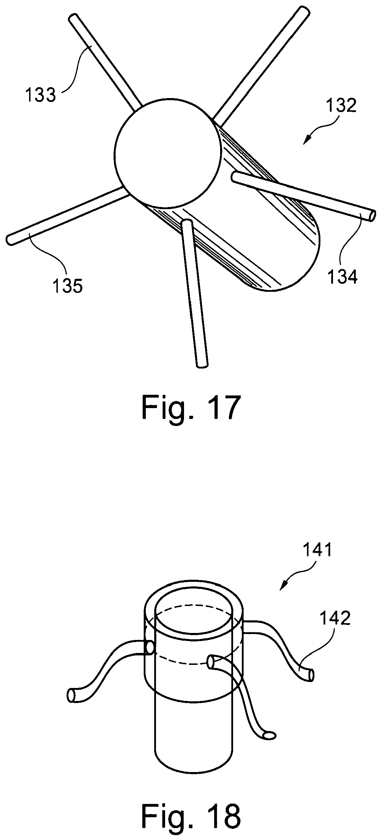

- Figur 17: eine schematische Darstellung einer Wickelvorrichtung mit horizontalen Stiften
- Figur 18: eine schematische Darstellung einer Wickelvorrichtung mit gebogenen Speichen und Wechselaufsatz zur Vergrößerung des Innendurchmessers der Spule
- Figur 19: eine schematische Darstellung einer Wickelvorrichtung mit vertikalen Stützstellen
- Figur 20: eine schematische Darstellung einer Wicklungsvorrichtung mit vertikalen Stützstellen, wobei diese geformt sind
- Figur 21: eine schematische Darstellung verschiedener HF Litzen

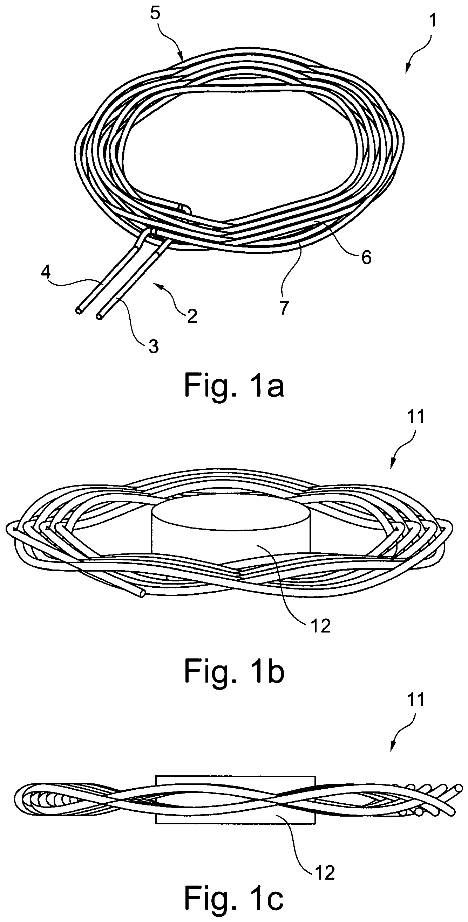

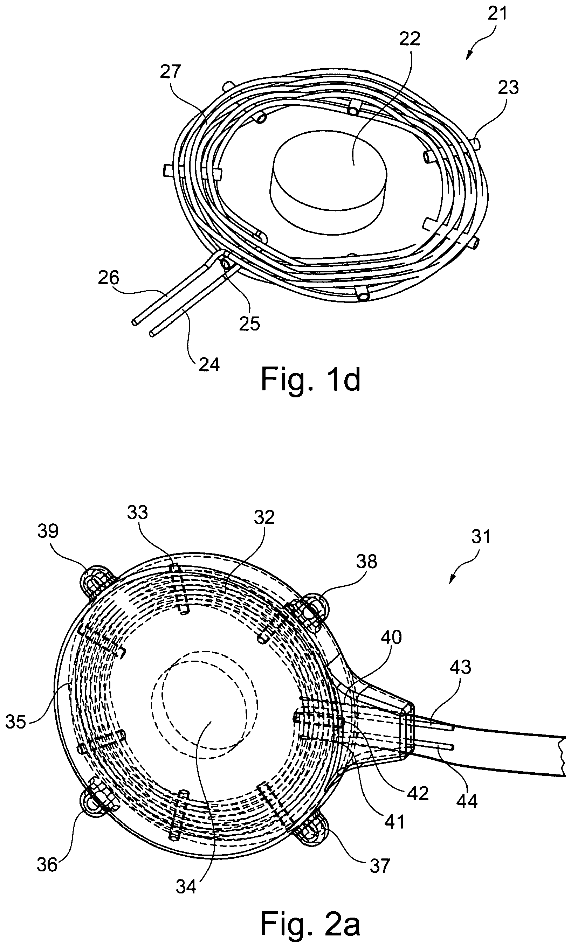

Die Wicklung (1) in Figur 1 wird aus dem die Leiter (2) geformt. Dieser weist ein inneres Leiterende (3) auf, mit dem die Wicklung (1) auf der Innenseite beginnt und dann bis zur Außenseite fortgeführt wird, wo der Leiter in ein äußeres Ende (4) endet. Durch die in den Kreuzungspunkten, wie beispielsweise Kreuzungspunkt (5) alternierende Wicklung des Leiters (2) entsteht die gewünschte Struktur. Die Kreuzungspunkte, wie der Kreuzungspunkt (5) bilden dabei eine Wicklungsebene. Vorliegend handelt es sich bei der Wicklung (1) um eine Wicklung mit sieben Kreuzungspunkten. Bei der Wicklung in Figur la sind keine Trägerröhrchen vorgesehen. Es kann sich beispielsweise um eine verklebte Variante handeln. In der Figur 1b und 1c ist eine Wicklung (11) dargestellt. Zusätzlich zu dieser ist eine Magnetscheibe (12) abgebildet. Diese bildet einen Teil einer Ausrichthilfe (weitere Teile nicht abgebildet).

Zusätzlich können zu der Wicklung (21), wie in Figur 1d abgebildet, neben der Magnetscheibe (22) auch Halteröhrchen, wie beispielsweise (23) vorgesehen sein. Der Innenleiter (24) ist durch eine der Öffnungen, in welche ein Halteröhrchen, hier (25), eingeführt ist, ebenfalls durchgeführt. Der Außenleiter (26) und der Innenleiter (24) sind nicht verdrillt. Die Wicklung weist sieben Kreuzungspunkte, wie Kreuzungspunkt (27) mit dazwischenliegenden Öffnungen auf, in die die Halteröhrchen, wie beispielsweise das Halteröhrchen (23) und das Halteröhrchen (25) eingeführt sind.

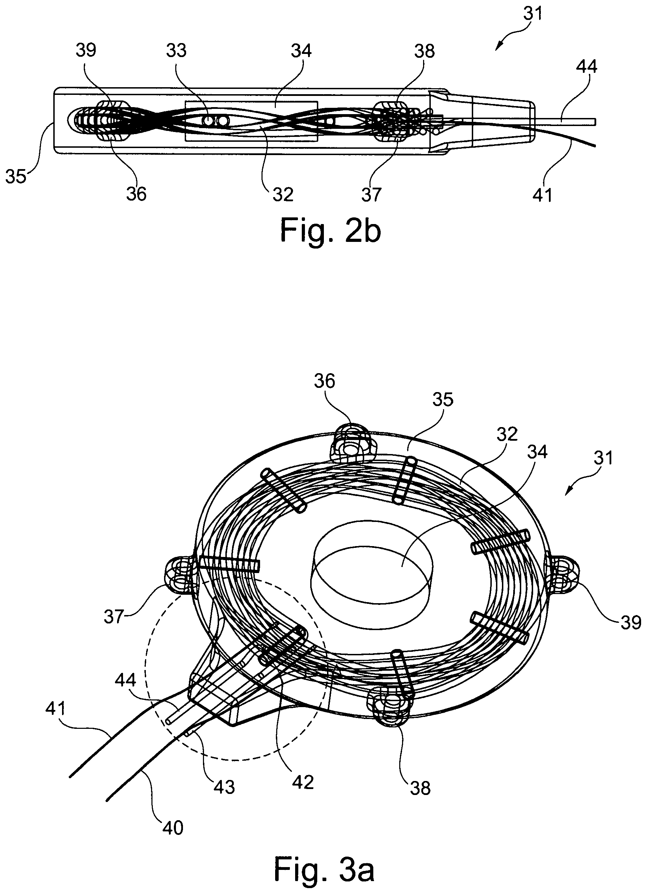

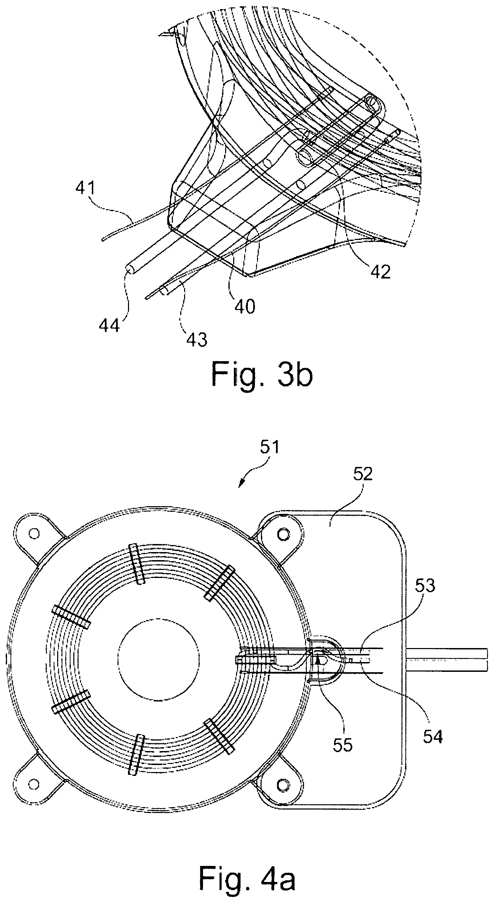

In einer Spule (31), wie in Figur 2a, 2b sowie 3a in und in der Detailansicht 3b dargestellt, wird die Wicklung (32) mit den Trägerröhrchen (33) sowie den Magneten (34) von einer Silikonumhüllung (35) umschlossen. Diese weist die Ösen (36), (37), (38) und (39) auf. Zusätzlich sind zwei Sensoren (40) und (41) eingebettet, die ebenfalls durch eine Öffnung der Wicklung geführt sind. Durch die Ösen (36), (37), (38) und (39) ist eine Befestigung der Spule (31) bei Implantation möglich. Die Einbringung der Sensoren (40) und (41) in die entsprechende Öffnung der Wicklung (32), zusammen mit dem Trägerröhrchen (42) sowie dem inneren Leiterende (43) und dem äußeren Leiterende (44) in die Öffnung ist in der Detaildarstellung 3b der Anschlussbereichs (45) besonders klar zu sehen.

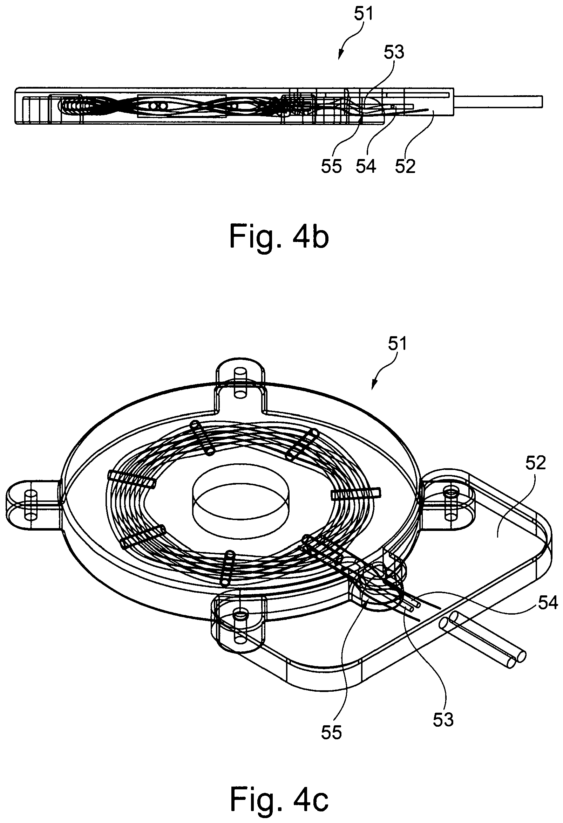

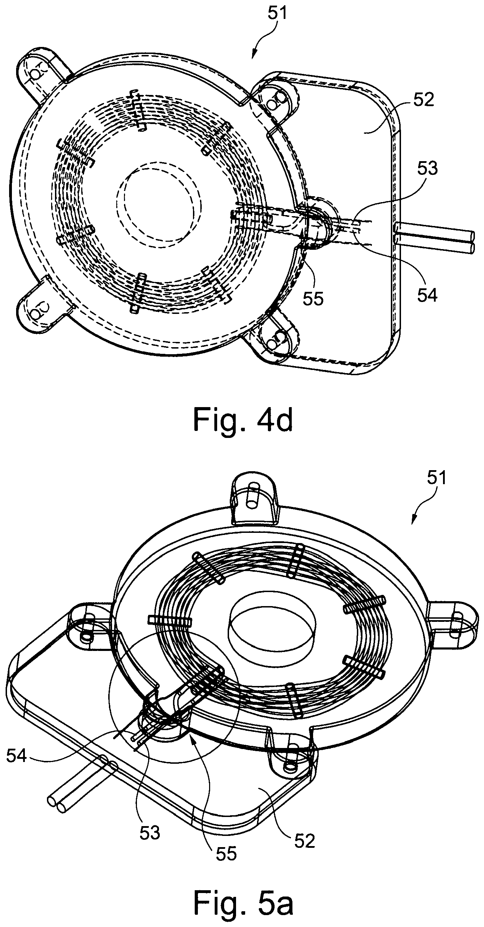

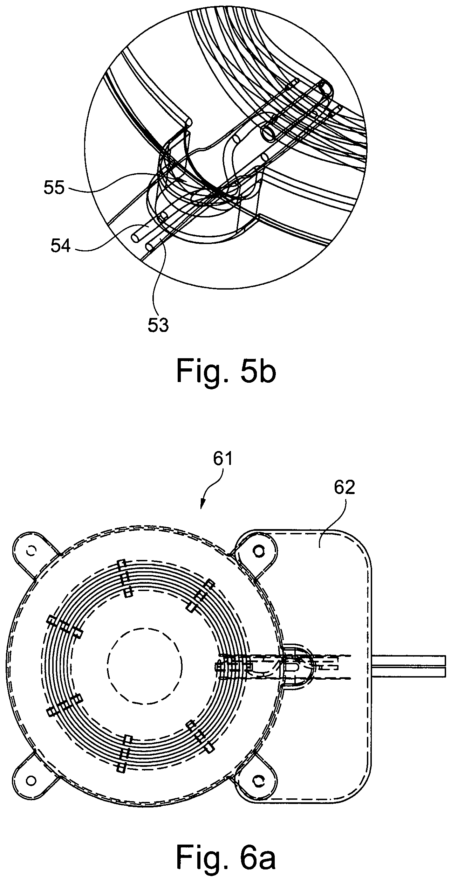

Die Einzelspule (51) in den Figuren 4a, b, c, d und Figur 5a und b ist mit einer Treiber- oder Empfangselektronik (52) versehen. Die Leiterenden 53 und 54 sind vor Eintritt in die Treiber- oder Empfangselektronik (52) an der Stelle (55) verdrillt. Dies ist besonders in der Detailansicht in Figur 5b erkennbar.

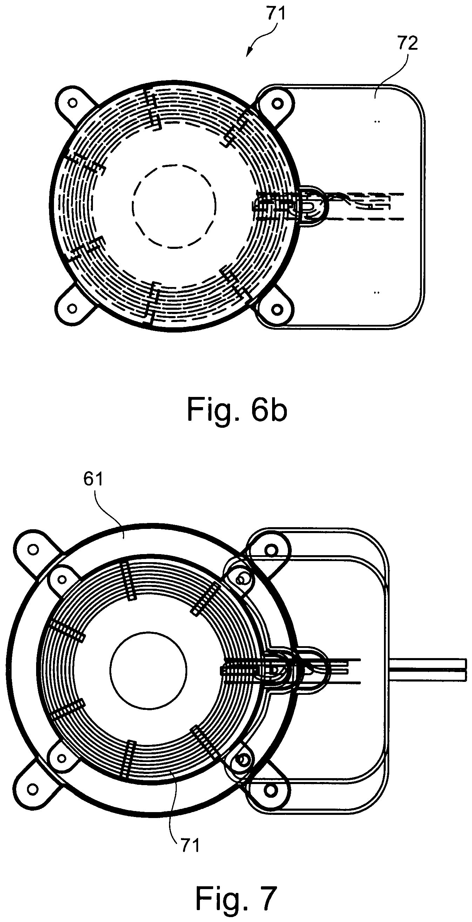

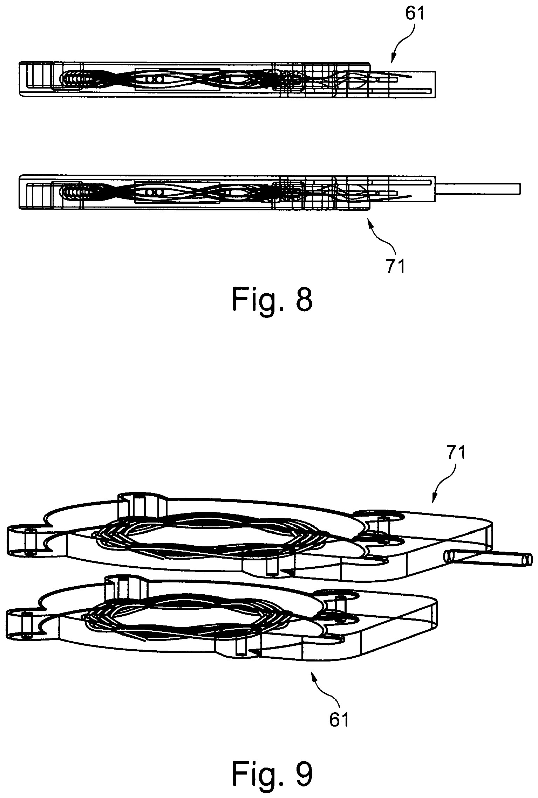

Ein Spulenpaar, wie in den Figuren 7, 8, 9 und 10 a und b abgebildet, setzt sich aus einer großen externen Primärspule (61) mit Treiberelektronik (62) sowie einer kleinen implantierbaren Spule (71) mit Empfangselektronik (72) zusammen.

Im Spulensystem (81) (Figur 11) versorgt eine Primärspule (82) mit Treiber (83) eine Sekundärspule (84), die ihrerseits zwei Verbraucher (85) und (86) versorgt.

Alternativ können auch zwei Leistungspfade (91) und (92) vorgesehen sein, in welchen jeweils eine Primärspule (93, 94) mit Treiberelektronik (95, 96) eine Sekundärspule (97, 98) mit entsprechendem Verbraucher (99, 100) versorgt (Figur 12).

Schließlich ist es jedoch auch möglich, in einem Spulensystem (101) mit einer Primärspule (102) und einem Treiber (103) zwei Sekundärspulen (104, 105) zu betreiben, die dann ihrerseits zwei Verbraucher (106, 107) versorgen (Figur 13). Dabei kann ein Leistungspfad für ein VAD genutzt werden, der andere zum Laden des internen Energiespeichers.

Ein Spulensystem mit einem aktiven medizinischen Implantat (111) ist in Figur 14 dargestellt. In einem solchen System wird durch eine Spannungsquelle (112) Energie in das System (111) eingespeist. Bei der Spannungsquelle (112) kann es sich um eine Gleichspannungsquelle in Form einer Batterie, eines Akkumulators oder ein Gleichspannungsnetzteil handeln. Über einen Stromrichter (113) und ein Primärkompensationsnetzwerk (114), vorzugsweise in Form eines Schaltkreises mit Serien- oder Parallelkapazität wird die Primärspule (115) betrieben. Bei dem Stromrichter (113) handelt es sich um einen geschalteten Stromtreiber für die Primärspule typischerweise um einen Leistungsverstärker der Klasse D oder E (switch-mode coil driver Class D or E). Über die Hautbarriere (116) wird so Energie, hier symbolisiert durch den Pfeil (117), auf die Sekundärspule (118) übertragen. Diese leitet die Energie über ein Sekundärkompensationsnetzwerk (119), beispielsweise einen Schaltkreis mit Serien- oder Parallelkapazität, einen Gleichrichter (120) mit Spannungsbegrenzer und/oder Filter und/oder Sicherheitselektronik bzw. Regelelektronik und eine Steuereinheit (121) an den Verbraucher (122) weiter. Bei dem Verbraucher kann es sich um ein Herzunterstützungssystem (VAD), Kunstherz (THD) oder andere aktive Implantate mit einem Leistungsbedarf von 5 - 50 Watt, mit oder ohne Sensoren handeln. Typischerweise weist ein solcher Verbraucher einen dreiphasigen bürstenlosen Gleichstrommotor auf.

Gleichzeitig ist ein Kreislauf für ein kontaktloses Laden vorgesehen, bestehend aus einem internen Energiespeicher (123) und einer Ladeeinheit (124). Des Weiteren ist ein kontaktloser Kommunikations- und Regelungskreislauf, der eine Rückkopplung ermöglicht, vorgesehen. In diesem werden Daten, symbolisiert durch den Pfeil (125) von einem Datensender (126), beispielsweise über AM- oder FM-Modulation auf die induktive Kopplung oder direkt auf einen Datenempfänger (127) übertragen und dort demoduliert. Eine extrakorporale Steuereinheit (128) wirkt dann entsprechend wieder auf den Stromrichter ein. Ein externer Zugriff wird durch das User-Interface (129) mit optischer und/oder akustischer Anzeige, die auch eine Alarmierungsmöglichkeit vorsehen kann, möglich.

In den Figuren 15 und 16 wird eine Wicklung (131) auf einem Hilfskörper (132) dargestellt. Dabei weist der Hilfskörper (132), wie in Figur 17 gut sichtbar, fünf zylindrische Speichen wie die Speichen (133, 134, 135) auf. Diese können lösbar, beispielsweise steck- oder schraubbar im Hilfskörper verankert sein. Beginnt man nun an dieser Stelle innen auf dem Hilfskörper (132) und führt einen Leiter (136) alternierend über- und unterhalb der Speichen, wie beispielsweise (133, 134, 135) des Hilfskörpers (132), so befinden sich diese in einer niedrigeren Position (137) und in einer höheren Position (138) zu einer durch die Kreuzungspunkte, wie beispielsweise Kreuzungspunkt (139) gebildeten Ebene, die auch die Bildebene in Figur 15 bildet. Die Abfolge der Positionen alterniert aufgrund der ungeraden Zahl der Speichen (133, 134, 135) des Hilfskörpers (132) sowohl entlang des Leiters (136) als auch radial von einer Wicklungsreihe zur nächsten. Die Berührpunkte zweier benachbarter Wicklungen bilden den Übergang zwischen den Zuständen in Wicklungsrichtung des Leiters und liegen in radialer Richtung auf Geraden wie (140).

Um andere Formgebungen zu erreichen, kann eine Wickelvorrichtung (141) gebogene Speichen, wie (152) aufweisen. Ein Wechselaufsatz (153) ermöglicht es den Innendurchmesser einer Spule (nicht abgebildet) zu vergrößern (Figur 18).

Die Herstellung einer Wicklung kann auch auf einer Wickelvorrichtung (151) mit vertikalen Stützstellen, wie beispielsweise (152) erfolgen (Figur 19).

Solche Stützstellen, wie beispielsweise (172) in einer Wickelvorrichtung (171) können auch gebogen sein (Figur 20).

## Patentansprüche

1. Spule zur induktiven transkutanen Energie- und/oder Datenübertragung für aktive medizinische Implantate, wobei Spulenwicklungen mit einem Leiter ausgeführt sind und in Spiralform um ein Zentrum verlaufen, zwischen zwei benachbarten vollständigen Wicklungen n Kreuzungspunkte existieren, die eine Wicklungsebene definieren, wobei n eine ungerade Zahl > 3 ist und die Lage des gewickelten Leiters hinsichtlich der Wicklungsebene vor und nach dem Kreuzungspunkt relativ zur Wicklungsebene zwischen einer niedrigen Position unterhalb der Wicklungsebene und einer hohen Position oberhalb der Wicklungsebene alterniert, **dadurch gekennzeichnet dass** die Spule eine Umhüllung aus einem biokompatiblen Material aufweist.

2. Spule insbesondere nach Anspruch 1, ***dadurch gekennzeichnet, dass*** radial benachbarte Kreuzungspunkte bezüglich des Zentrums stets den gleichen Winkel einschließen.

3. Spule nach einem der Ansprüche 1 oder 2, ***dadurch gekennzeichnet, dass*** an einer Stelle eines größten Abstandes des Leiters zur Wicklungsebene eine Leiteraußenseite einen Abstand von mindestens 0,5, insbesondere von mindestens 1,0 des Leitungsdurchmessers zur Wicklungsebene aufweist.

4. Spule nach einem der vorhergehenden Ansprüche, ***dadurch gekennzeichnet, dass*** durch das Alternieren zwischen der niedrigen und der hohen Position Hohlräume entstehen, die durchgängig von der Spulenmitte radial nach außen verlaufen.

5. Spule nach Anspruch 4, ***dadurch gekennzeichnet, dass*** ein inneres Leiterende durch einen Hohlraum nach außen geführt ist.

6. Spule nach Anspruch 4 oder 5, ***dadurch gekennzeichnet, dass*** durch einen Hohlraum ein Sensor, insbesondere ein Temperatursensor geführt ist.

7. Spule nach einem der Ansprüche 4 bis 6, ***dadurch gekennzeichnet, dass*** durch einen Hohlraum eine Antenne geführt ist.

8. Spule nach einem der Ansprüche 4 bis 7, ***dadurch gekennzeichnet, dass*** durch einen Hohlraum ein gerades oder gebogenes Stützelement geführt ist.

9. Spule nach einem der vorhergehenden Ansprüche, ***dadurch gekennzeichnet, dass*** das biokompatible Material ein Silikon oder ein anderer langzeitgetesteter biokompatibler Kunststoff, wie beispielsweise ein Polymer wie Polyurethan, PTFE, PEEK oder ein anderer temperaturbeständiger Thermoplast ist.

10. Spulenpaar zur induktiven transkutanen Übertragung von Leistung zur Energieversorgung aktiver medizinischer Implantate mit einer implantierbaren Sekundärspule und einer externen Primärspule, wobei eine der Spulen, insbesondere die implantierbare Sekundärspule eine Spule gemäß den Ansprüchen 1 bis 9 ist.

11. Spulenpaar nach Anspruch 10, ***dadurch gekennzeichnet, dass*** das Spulenpaar eine Ausrichthilfe aufweist.

12. Spulenpaar nach einem der vorhergehenden Ansprüche, ***dadurch gekennzeichnet, dass*** es eine Sicherheitselektronik aufweist, eine Fremdobjekterkennung und/oder eine analoge Testanfrage und/oder eine digitale Identifizierung und/oder einen Überstromschutz und/oder eine Spannungsklemme und/oder eine Temperaturerfassung und/oder einen Hochspannungstransientenschutz (high voltage surge stopper) und/oder einen ESD-Schutz und/oder eine Ausschaltmöglichkeit und/oder einen Standby-Modus des aktiven Synchrongleichrichters aufweist.

13. Spulenpaar nach einem der Ansprüche 10 bis 12, ***dadurch gekennzeichnet, dass*** das Spulenpaar Leistungen in der Größenordnung von 5 - 50 Watt, insbesondere von 5 - 40 Watt, insbesondere von 10 - 30 Watt überträgt.

14. Spulensystem mit einem Spulenpaar nach einem der Ansprüche 10 bis 13, ***dadurch gekennzeichnet, dass*** es ein weiteres Spulenpaar aufweist.

15. Aktives medizinisches Implantat, insbesondere Herzunterstützungssystem, insbesondere mit einem Leistungsbedarf von 5 *-* 50 Watt mit einer Spule insbesondere nach einem der Ansprüche 1 bis 9 oder einem Spulenpaar nach einem der Ansprüche 10 bis 14 oder einem Spulensystem nach Anspruch 14.

## Claims

1. A bobbin for inductive transcutaneous energy and/or data transmission for active medical implants, wherein bobbin windings are implemented as conductors wound around a centre in the form of a spiral, n intersecting points defining a winding plane exist between two adjacent complete windings, wherein n is an odd number > 3, and the position of the wound conductor, as regards the winding plane in front of and behind the intersecting point, alternates relative to the winding plane between a low position below the winding plane and a high position above the winding plane, ***characterised in that*** the bobbin comprises an encasement from a bio-compatible material.

2. The bobbin in particular according to claim 1, ***characterised in that*** radially adjacent intersecting points, with respect to the centre, always enclose the same angle.

3. The bobbin according to one of claims 1 or 2, ***characterised in that*** at a point of a largest distance of the conductor from the winding plane, a conductor outside comprises a distance from the winding plane of at least 0.5, in particular of at least 1.0 of the line diameter.

4. The bobbin according to one of the preceding claims, ***characterised in that*** the alternating between the low and the high position causes cavities to be created, which extend continuously radially outward from the centre of the bobbin.

5. The bobbin according to claim 4, ***characterised in that*** an inner conductor end is run through a cavity to the outside.

6. The bobbin according to claim 4 or 5, ***characterised in that*** one cavity has a sensor, in particular a temperature sensor, extending through it.

7. The bobbin according to one of claims 4 to 6, ***characterised in that*** one cavity has an aerial extending through it.

8. The bobbin according to one of claims 4 to 7, ***characterised in that*** one cavity has a straight or bent supporting element extending through it.

9. The bobbin according to one of the preceding claims, ***characterised in that*** the bio-compatible material is a silicone or another time-tested bio-compatible plastic, for example a polymer such as polyurethane, PTFE, PEEK or another temperature-resistant thermoplastic.

10. A bobbin pair for inductive transcutaneous power transmission for supplying energy to active medical implants having an implantable secondary bobbin and an external primary bobbin, wherein one of the bobbins, in particular the implantable secondary bobbin, is a bobbin according to claims 1 to 9.

11. The bobbin pair according to claim 10, ***characterised in that*** the bobbin pair comprises an alignment aid.

12. The bobbin pair according to one of the preceding claims, ***characterised in that*** it comprises a safety electronics, comprises a foreign object detection and/or an analogous test enquiry and/or a digital identification and/or an overcurrent protection and/or a voltage terminal and/or a temperature detection and/or a high-voltage surge stopper and/or an ESD-protection and/or a switch-off option and/or a standby mode of the active synchronous rectifier.

13. The bobbin pair according to one of claims 10 to 12, ***characterised in that*** the bobbin pair transmits power of a magnitude of 5 - 50 Watts, in particular 5 - 40 watts, in particular 10 - 30 watts.

14. A bobbin system with a bobbin pair according to one of claims 10 to 13, ***characterised in that*** it comprises a further bobbin pair.

15. An active medical implant, in particular heart support system, in particular with a power requirement of 5 - 50 watts with a bobbin in particular according to one of claims 1 to 9 or a bobbin pair according to one of claims 10 to 14 or a bobbin system according to claim 14.

## Revendications

1. Bobine pour la transmission transcutanée inductive d'énergie et/ou de données pour un implant médical actif, dans lequel des enroulements de bobine sont conçus avec un conducteur et s'étendent en forme de spirale autour d'un centre, entre deux enroulements complets voisins existent n points de croisement, qui définissent un plan d'enroulement, dans lequel n est un nombre impair > 3 et la position du conducteur enroulé par rapport au plan d'enroulement avant et après le point de croisement par rapport au plan d'enroulement alterne entre une position basse au-dessous du plan d'enroulement et une position haute au-dessus du plan d'enroulement, ***caractérisé en ce que*** la bobine présente une enveloppe constituée d'un matériau biocompatible.

2. Bobine notamment selon la revendication 1, ***caractérisé en ce que*** des points de croisement radiaux voisins définissent constamment le même angle par rapport au centre.

3. Bobine selon une des revendications 1 ou 2, ***caractérisé en ce que*** à un emplacement d'un grand espacement du conducteur par rapport au plan d'enroulement, un côté extérieur du conducteur présente un espacement d'au moins 0,5, notamment d'au moins 1,0 du diamètre de ligne par rapport au plan d'enroulement.

4. Bobine selon une des revendications précédentes, ***caractérisé en ce que*** en alternant entre la position basse et la position haute des espaces creux sont générés, qui s'étendent en continu radialement vers l'extérieur à partir du milieu de bobine.

5. Bobine selon la revendication 4, ***caractérisé en ce que*** une extrémité de conducteur intérieur est guidée vers l'extérieur à travers un espace creux.

6. Bobine selon la revendication 4 ou 5, ***caractérisé en ce que*** un capteur, notamment un capteur de température est guidé à travers un espace creux.

7. Bobine selon une des revendications 4 à 6, ***caractérisé en ce que*** une antenne est guidée à travers un espace creux.

8. Bobine selon une des revendications 4 à 7, ***caractérisé en ce que*** un élément d'appui droit ou arqué est guidé à travers un espace creux.

9. Bobine selon une des revendications précédentes, ***caractérisé en ce que*** le matériau biocompatible est un silicone ou un autre plastique biocompatible testé sur une longue durée, comme par exemple un polymère comme du polyuréthane, PTFE, PEEK ou un autre thermoplaste résistant aux températures.

10. Paire de bobines pour la transmission transcutanée inductive de puissance à des fins d'alimentation électrique d'implants médicaux actifs avec une bobine secondaire pouvant être implantée et une bobine primaire externe, dans lequel une des bobines, notamment la bobine secondaire implantable est une bobine selon les revendications 1 à 9.

11. Paire de bobines selon la revendication 10, ***caractérisé en ce que*** la paire de bobine présente une aide à l'alignement.

12. Paire de bobines selon une des revendications précédentes, ***caractérisée en ce que*** elle présente une électronique de sécurité, une reconnaissance d'objet étranger et/ou une interrogation de test analogique et/ou une identification numérique et/ou une protection contre les surintensités et/ou une borne pour la tension et/ou une détection de température et/ou une protection contre les surtensions transitoires (high voltage surge stopper) et/ou une protection ESD et/ou une possibilité de mise hors tension et/ou un mode de veille d'un redresseur synchrone actif.

13. Paire de bobines selon une des revendications 10 à 12, ***caractérisé en ce que*** la paire de bobines transmet des puissances dans l'ordre de grandeur de 5-50 Watt, notamment de 5-40 Watt, notamment de 10-30 Watt.

14. Système de bobines avec une paire de bobines selon une des revendications 10 à 13, ***caractérisé en ce que*** il présente une paire de bobines supplémentaire.

15. Implant médical actif, notamment système d'assistance cardiaque, notamment avec un besoin en puissance de 5-50 Watt avec une bobine notamment selon une des revendications 1 à 9 ou une paire de bobines selon une des revendications 10 à 14 ou un système de bobines selon la revendication 14.
